# EUROPEAN PATENT APPLICATION

(11) **EP 4 411 139 A1**
(43) Date of publication of application: **07.08.2024**
(21) Application number: 22897034.9
(22) Date of filing: 22.04.2022
(51) Int. Cl.: F04B 43/04

(54) **MICROFLUIDIC APPARATUS AND ELECTRONIC DEVICE**

(30) Priority: 25.11.2021 CN 202122916844 U
(71) Applicant: Huawei Technologies Co., Ltd., Shenzhen, Guangdong 518129 (CN)
(72) Inventor: LI, Shiguang, Shenzhen, Guangdong 518129 (CN); WU, Nan, Shenzhen, Guangdong 518129 (CN); WANG, Xiaodong, Shenzhen, Guangdong 518129 (CN); XIONG, Linqiang, Shenzhen, Guangdong 518129 (CN); ZHENG, Jianfeng, Shenzhen, Guangdong 518129 (CN); CHENG, Yupeng, Shenzhen, Guangdong 518129 (CN); ZHANG, Yunjie, Shenzhen, Guangdong 518129 (CN); HE, Fu, Shenzhen, Guangdong 518129 (CN)
(74) Representative: Isarpatent
(86) International application number: PCT/CN2022/088374
(87) International publication number: WO 2023/092934

(57) **Abstract**

A micro fluid control apparatus (100) and an electronic device (1) are provided. The micro fluid control apparatus (100) includes an oscillator layer (20) and a resonance layer (10) that are stacked. A vibration portion (21) of the oscillator layer (20) is connected to a fastening portion (22) by using a connection portion (23). The vibration portion (21) can vibrate. The micro fluid control apparatus (100) further includes a bonding layer (30) and a support structure (12). The bonding layer (30) is located between the fastening portion (22) and the resonance layer (10), so that the resonance layer (10) is connected to the oscillator layer (20) by using the bonding layer (30). The support structure (12) is also located between the fastening portion (22) and the resonance layer (10), so that a spacing is reserved between the resonance layer (10) and the oscillator layer (20). In this way, the support structure (12), the oscillator layer (20), and the resonance layer (10) form, in an enclosing manner, a chamber for accommodating fluid and allowing the fluid to pass through, to control a flow of the fluid. The spacing is formed between the oscillator layer and the resonance layer by using a support height of the support structure, and a height and a position of the support structure are easy to control, so that a height of the spacing is accurately controlled. This reduces or avoids an excessively large or excessively small height of the spacing, ensures consistency of the height of the spacing, and improves performance of the micro fluid control apparatus.

## Description

This application claims priority to Chinese Patent Application No. 202122916844.9, filed with the China National Intellectual Property Administration on November 25, 2021, and entitled "MICRO PIEZOELECTRIC PUMP AND ELECTRONIC DEVICE", which is incorporated herein by reference in its entirety.

### TECHNICAL FIELD

This application relates to the field of microfluidics technologies, and in particular, to a micro fluid control apparatus and an electronic device.

### BACKGROUND

Microfluidics (Microfluidics) means the science and technology involved in a system that processes or manipulates micro fluid by using a micro pipe (for example, tens to hundreds of micrometers in size), and has gained wide attention in the fields of biomedical research and micro electronic device technologies currently. For example, an electronic device having a wristband, such as a smartwatch/band, implements blood pressure detection and other functions by using a micro piezoelectric pump. Specifically, for example, an air bag and the micro piezoelectric pump may be disposed in the wristband. During wearing, the air bag is in contact with a body surface and is inflated by using the micro piezoelectric pump, the air bag applies pressure to the body surface, a change trend of an amplitude of an oscillation wave of an arterial vessel in a process in which the air bag applies the pressure and performs blocking is measured, and systolic blood pressure and diastolic blood pressure are calculated based on a pressure change in the air bag. In this way, a function of medical-level blood pressure detection of a wearable device is implemented.

Currently, a micro fluid control apparatus usually includes an oscillator layer and a resonance layer. The oscillator layer is provided with a vibration portion. There is a spacing between the oscillator layer and the resonance layer, to reserve space for the vibration portion to vibrate. In this way, a flow rate and pressure of fluid in the spacing are changed, and flow rate and back pressure features required by the micro fluid control apparatus are provided. For example, a micro piezoelectric pump may include a resonance layer and an oscillator layer that are stacked. A spacing needs to be reserved between the resonance layer and the oscillator layer, to form an inhalation chamber. A vibration portion of the oscillator layer vibrates up and down relative to the resonance layer, to change size states of the spacing and the inhalation chamber. In this way, air is inhaled into the inhalation chamber and is discharged, to pump the air. A size of the spacing between the oscillator layer and the resonance layer directly affects performance of the micro fluid control apparatus, for example, directly affects an air pumping capacity of the piezoelectric pump. To reserve the spacing between the oscillator layer and the resonance layer, microbead colloid is usually used as a support for the spacing. Specifically, micro steel beads are evenly mixed into colloid. Then, the colloid including microbeads is disposed on the resonance layer in a dispensing manner, and a fastening portion of the oscillator layer is disposed on the colloid mixed with the microbeads, so that the oscillator layer is connected to the resonance layer by using the colloid mixed with the microbeads. In addition, the microbeads are located between the oscillator layer and the resonance layer, to form the required spacing.

However, because distribution of the microbeads in the colloid is uncontrollable, a height of the spacing between the oscillator layer and the resonance layer is uncontrollable, that is, the height of spacing is difficult to remain consistent. This reduces consistency of the flow rate and pressure of the fluid in the spacing, and affects performance of the micro fluid control apparatus.

### SUMMARY

This application provides a micro fluid control apparatus and an electronic device, to resolve a problem that performance of an existing micro fluid control apparatus is affected due to poor consistency of a height of a spacing between an oscillator layer and a resonance layer in the existing micro fluid control apparatus.

A first aspect of this application provides a micro fluid control apparatus. The micro fluid control apparatus includes: an oscillator layer and a resonance layer that are stacked. The oscillator layer includes a vibration portion, a fastening portion, and a connection portion. The fastening portion encloses an outer periphery of the vibration portion. The vibration portion is connected to the fastening portion by using the connection portion. A through opening for fluid to pass through is provided between the vibration portion and the fastening portion.

The micro fluid control apparatus further includes a bonding layer and a support structure. The bonding layer is located between the fastening portion and the resonance layer. The bonding layer surrounds the outer periphery of the vibration portion. The fastening portion is connected to the resonance layer by using the bonding layer. In this way, the resonance layer and the oscillator layer are assembled and fastened.

The support structure is located between the resonance layer and the fastening portion, so that a spacing is formed between the oscillator layer and the resonance layer. In this way, space for the vibration portion to vibrate is reserved. In addition, the support structure, the oscillator layer, and the resonance layer can form, in an enclosing manner, a chamber that can be configured to accommodate fluid and allow the fluid to pass through. An inlet for the fluid to enter the chamber may be provided on the resonance layer. A through opening for the fluid to flow out of the chamber may be provided on the oscillator layer. When the vibration portion vibrates at a high frequency relative to the resonance layer, sizes of the spacing between the oscillator layer and the resonance layer and the formed chamber change. In this way, a flow rate and pressure of the fluid located in the chamber are changed, and the fluid can be pumped into the chamber from the inlet and pumped out of the chamber from the through opening on the oscillator layer, to control a flow of the fluid and provide a required flow rate and back pressure. For example, the micro fluid control apparatus is a micro piezoelectric pump. When the vibration portion of the oscillator layer vibrates at a high frequency, an air flow can enter an inhalation chamber from an air intake opening and be pumped out from an air discharge opening, to pump air.

In addition, the support structure is located in a radial direction of the bonding layer. Two ends of the bonding layer are respectively connected to the oscillator layer and the resonance layer, so that the resonance layer is fixedly connected to the oscillator layer. Two ends of the support structure are also respectively connected to the oscillator layer and the resonance layer. The support structure provides a good support function. The bonding layer does not exist between the oscillator layer and the support structure or between the support structure and the resonance layer. The spacing is reserved between the oscillator layer and the resonance layer by using a support height of the support structure. In other words, a height of the spacing is determined by a height of the support structure. The height of the support structure, a disposing position of the support structure, and the like are easily controlled. For example, a preparation process condition or the like of the support structure can be adjusted, to adjust the height and the position of the support structure. In this way, the height of the spacing between the oscillator layer and the resonance layer is accurately controlled, and the height of the spacing can be accurately remained at a required specified value. This can avoid a problem that the flow rate and back pressure of the fluid are excessively small due to an excessively large height of the spacing, and ensure performance of the micro fluid control apparatus. In addition, this can avoid a problem of collision of the resonance layer and the oscillator layer during vibration due to an excessively small height of the spacing, reduce or avoid problems of an abnormal sound of impact and poor reliability due to the impact, and improve a yield rate of the micro fluid control apparatus and user experience.

In addition, this can ensure consistency of the height of the spacing between the resonance layer and the oscillator layer, improve control performance on the fluid, and improve performance of the micro fluid control apparatus. For example, the micro fluid control apparatus is a micro piezoelectric pump. The height of the spacing between the oscillator layer and the resonance layer is consistent. This helps improve consistency of an air flow and back pressure of the micro piezoelectric pump, and improve air pumping performance of the micro piezoelectric pump.

In a possible implementation, the support structure is connected to the resonance layer, and the oscillator layer abuts against the support structure. The support structure is disposed on the resonance layer, so that difficulty in molding the oscillator layer whose original structure is complex can be reduced. This helps implement mass production.

In a possible implementation, a protrusion is provided on a surface that is of the resonance layer and that faces the oscillator layer, to form the support structure. The support structure and the resonance layer are integrally molded. Because the height of the support structure is usually required to be at a micrometer level, using an integrally molding manner can facilitate molding and assembling of the support structure, reduce machining and assembling difficulty, and help improve control precision of the height of the spacing.

In a possible implementation, the support structure and the resonance layer are separately molded. The support structure is disposed on a surface that is of the resonance layer and that faces the oscillator layer. This improves disposition flexibility of the support structure and enriches application scenarios of the micro fluid control apparatus.

In a possible implementation, the support structure is a plating layer. For example, the support structure may be a metal plating layer. A molding manner is simple and fast, and has high feasibility.

In a possible implementation, the support structure is disposed on the resonance layer by using the bonding layer. In other words, when the resonance layer is connected to the oscillator layer by using the bonding layer, the support structure is fastened to the resonance layer. This facilitates implementation and helps reduce costs.

In a possible implementation, the support structure is a metal wire. Inner and outer diameters of the metal wire have high controllability. This helps further improve the consistency of the height of the spacing.

In a possible implementation, the support structure is located on a circumferential outer side of the bonding layer. The resonance layer and the support structure located on the resonance layer may form glue accommodating space, and the bonding layer may be disposed in the glue accommodating space. In this way, the resonance layer, the oscillator layer, and the support structure can be connected by using the bonding layer. This helps ensure stability of the micro fluid control apparatus.

In a possible implementation, a protrusion portion is provided on a surface that is of the fastening portion and that faces the resonance layer. The protrusion portion, the resonance layer, and the support structure jointly form accommodating space. The bonding layer is located in the accommodating space. In this way, the bonding layer can be conveniently disposed, and contact areas between the resonance layer and the oscillator layer, and the bonding layer can also be increased. This enhances connection fastness between the resonance layer and the oscillator layer and improves sealing performance of the chamber.

In a possible implementation, there are at least two support structures, and at least one of the support structures is located on a circumferential inner side of the bonding layer. The support structure located on the inner side may block and limit the bonding layer. This reduces or avoids problems such as glue overflow caused by diffusion of colloid toward the vibration portion of the oscillator layer in a process of forming the bonding layer in a dispensing manner, and ensures vibration performance of the vibration portion.

In a possible implementation, there is a gap between the at least two support structures, to form an accommodating groove, and the bonding layer is located in the accommodating groove. This can facilitate dispensing of colloid, to form the bonding layer, improve stability of the colloid, and effectively reduce overflow of the colloid. In addition, this also helps increase contact areas between the support structure and the oscillator layer, and the bonding layer, and ensure bonding fastness between the resonance layer and the oscillator layer.

In a possible implementation, the support structure is in a ring shape. The oscillator layer can be evenly supported, and the consistency of the height of the spacing can be further ensured.

In a possible implementation, the support structure is in a closed ring shape. The consistency of the height of the spacing can be further improved, and a molding manner is simple and easy to operate.

In a possible implementation, the support structure includes a plurality of support parts, and the plurality of support parts are spaced from each other and evenly disposed in a surrounding manner, to form the ring shape. This reduces costs and ensures evenness of disposition of the support parts on the resonance layer.

In a possible implementation, the support part includes at least two sub-support bodies, and two adjacent sub-support bodies are spaced from each other. Materials of the support parts can be further reduced. This helps further reduce the costs and facilitates a weight reduction design of the micro fluid control apparatus.

In a possible implementation, a cross-sectional shape of the support part includes at least a long strip shape, a cylindrical shape, an arc shape, and a square shape.

In a possible implementation, a height range of the support structure is 5 µm to 12 µm. The height of the spacing within the range is not too large to cause an excessively small flow rate and pressure of the fluid in the chamber, and is not too small to cause collision of the oscillator layer and the resonance layer during vibration and problems of an abnormal sound of impact and poor reliability.

In a possible implementation, the micro fluid control apparatus is a micro piezoelectric pump, and can achieve good air pumping performance.

A second aspect of this application provides an electronic device, including at least a housing and any one of the foregoing micro fluid control apparatuses. The micro fluid control apparatus is disposed in the housing.

The micro fluid control apparatus is included, and the micro fluid control apparatus can accurately control the spacing between the oscillator layer and the resonance layer of the micro fluid control apparatus, to effectively improve control of the micro fluid control apparatus on a flow rate, pressure, and the like of fluid. In this way, the micro fluid control apparatus has good control performance on a flow of the fluid. This improves performance of the electronic device and improves user experience.

A third aspect of this application provides a micro piezoelectric pump, including a quasi-valve resonance diaphragm and a piezoelectric vibration diaphragm that are stacked. The piezoelectric vibration diaphragm includes a vibration portion and a plate portion that encloses an outer periphery of the vibration portion. The plate portion is connected to the vibration portion by using a connection portion. An air discharge opening is provided on the connection portion. An air intake opening is provided at a middle part of the quasi-valve resonance diaphragm.

The micro piezoelectric pump further includes a bonding layer located between the plate portion and the quasi-valve resonance diaphragm. The bonding layer surrounds the outer periphery of the vibration portion. The quasi-valve resonance diaphragm is connected to the piezoelectric vibration diaphragm by using the bonding layer.

The micro piezoelectric pump further includes at least one support structure. The support structure is located between the quasi-valve resonance diaphragm and the plate portion, so that a spacing is formed between the piezoelectric vibration diaphragm and the quasi-valve resonance diaphragm. The at least one support structure is located on an inner side of the bonding layer.

In a possible implementation, the support structure is protruded on a surface that is of the quasi-valve resonance diaphragm and that faces the piezoelectric vibration diaphragm. The piezoelectric vibration diaphragm abuts against the support structure.

In a possible implementation, the support structure is in a ring shape. Each support structure includes a plurality of support parts. The plurality of support parts are spaced from each other and evenly disposed in an enclosing manner, to form the ring shape.

In a possible implementation, the support structure is in a closed ring shape.

In a possible implementation, the quasi-valve resonance diaphragm is provided with at least two support structures.

In two adjacent support structures, one support structure is located on an inner side of the other support structure. There is a gap between the two adjacent support structures. The bonding layer is located in the gap.

In a possible implementation, the support part includes at least two sub-support bodies, and two adjacent sub-support bodies are spaced from each other.

In a possible implementation, a cross-sectional shape of the support part includes at least a long strip shape, a cylindrical shape, an arc shape, and a square shape.

In a possible implementation, the support structure includes a plating layer.

In a possible implementation, the support structure includes a metal wire.

In a possible implementation, a height range of the support structure is 5 µm to 12 µm.

In a possible implementation, the micro piezoelectric pump further includes an air intake layer and an air intake channel that are stacked. The air intake channel is located between the air intake layer and the quasi-valve resonance diaphragm.

An air intake hole is provided on the air intake layer. The air intake hole communicates with the air intake opening through the air intake channel.

In a possible implementation, the micro piezoelectric pump further includes a front chamber structure layer, an air leakage diaphragm, an air vent channel, and an air vent layer that are sequentially stacked. The piezoelectric vibration diaphragm is located between the quasi-valve resonance diaphragm and the front chamber structure layer.

The air discharge opening communicates with the front chamber structure layer. The front chamber structure layer communicates with the air leakage diaphragm. The air leakage diaphragm communicates with the air vent layer through the air vent channel. An air vent hole is provided on the air vent layer.

A fourth aspect of this application provides an electronic device, including at least a housing, an air bag, and any one of the foregoing micro piezoelectric pumps.

The air bag and the micro piezoelectric pump are disposed in the housing. The micro piezoelectric pump communicates with the air bag.

In a possible implementation, the housing includes a wristband, and the air bag is disposed in the wristband.

### BRIEF DESCRIPTION OF DRAWINGS

FIG. 1 is a schematic diagram of a structure of an electronic device according to an embodiment of this application;
FIG. 2 is a schematic diagram of a structure of a micro fluid control apparatus according to an embodiment of this application;
FIG. 3 is a schematic diagram of a partial cross-sectional structure of a micro fluid control apparatus according to an embodiment of this application;
FIG. 4 is a schematic diagram of a structure of another micro fluid control apparatus from a view angle according to an embodiment of this application;
FIG. 5 is a schematic diagram of a structure of another micro fluid control apparatus from another view angle according to an embodiment of this application;
FIG. 6 is a schematic diagram of a cross-sectional structure of existing assembly of a resonance layer and an oscillator layer;
FIG. 7 is a schematic diagram of a cross-sectional structure of assembly of a resonance layer and an oscillator layer according to an embodiment of this application;
FIG. 8 is a schematic top view of assembly of a resonance layer and a bonding layer according to an embodiment of this application;
FIG. 9 is a schematic diagram of a structure of an oscillator layer according to an embodiment of this application;
FIG. 10 is a schematic top view of a structure of a resonance layer according to an embodiment of this application;
FIG. 11 is a schematic diagram of a structure of another resonance layer according to an embodiment of this application;
FIG. 12 is a schematic diagram of splitting of an oscillator layer and a resonance layer according to an embodiment of this application;
FIG. 13 is a schematic diagram of a structure obtained by assembling an oscillator layer and a resonance layer according to an embodiment of this application;
FIG. 14 is a schematic diagram of fitting of a resonance layer and an oscillator layer obtained when a micro fluid control apparatus is in a non-working state according to an embodiment of this application;
FIG. 15 is a schematic diagram of fitting of a resonance layer and an oscillator layer obtained when a micro fluid control apparatus is in a first working state according to an embodiment of this application;
FIG. 16 is a schematic diagram of fitting of a resonance layer and an oscillator layer obtained when a micro fluid control apparatus is in a second working state according to an embodiment of this application;
FIG. 17 is a schematic diagram of a cross-sectional structure obtained by fitting another oscillator layer with another resonance layer according to an embodiment of this application;
FIG. 18 is a schematic diagram of a partial cross-sectional structure obtained by fitting still another oscillator layer with still another resonance layer according to an embodiment of this application;
FIG. 19 is a schematic diagram of a cross-sectional structure obtained by fitting yet another oscillator layer **with** yet another resonance layer according to an embodiment of this application;
FIG. 20 is a schematic diagram of a support structure on a resonance layer according to an embodiment of this application; and
FIG. 21 is a schematic diagram of molding of another support structure according to an embodiment of this application.

### Reference numerals:

1: electronic device; 111: wristband; 112: watch face;
100: micro fluid control apparatus; 10: resonance layer; 11: air intake opening;
12: support structure; 121: support part; 121a: sub-support body;
20: oscillator layer; 21: vibration portion; 22: fastening portion;
221: protrusion portion; 23: connection portion; 231: air discharge opening;
30: bonding layer; 40: air intake layer; 41: air intake hole;
50: air intake channel; 60: front chamber structure layer; 70: air leakage diaphragm;
80: air vent channel; 90: air vent layer; 91: air vent hole;
110: spacing; 120: inhalation chamber; 130: oscillator electrode;
140: gap.

### DESCRIPTION OF EMBODIMENTS

Terms used in embodiments of this application are merely used to explain specific embodiments of this application, but are not intended to limit this application.

An embodiment of this application provides a micro fluid control apparatus, which may be used in a microfluidics system, to control a flow of fluid such as air or liquid. The micro fluid control apparatus may be a micro air pump, for example, a micro piezoelectric pump, or the micro fluid control apparatus may be a blower, or the micro fluid control apparatus may be another type of apparatus for controlling a flow of fluid.

An embodiment of this application further provides an electronic device including the micro fluid control apparatus. The electronic device may be a portable terminal device such as a wearable device. For example, the wearable device may be an augmented reality (Augmented Reality, AR) device, a virtual reality (Virtual Reality, VR) device, or an artificial intelligence (Artificial Intelligence, AI) device. The wearable electronic device may implement a functional characteristic of the wearable electronic device through wearable performance. For example, the micro fluid control apparatus is a micro air pump. The wearable electronic device includes the micro air pump. The wearable electronic device may be in contact with a human body through wearing, and control a flow rate and pressure of air by using the micro air pump, to pump air. In this way, blood pressure of a user can be detected.

For example, the electronic device may be a band, a watch, glasses, a helmet, a headband, or another smart clothing, tool, or accessory. Alternatively, the electronic device may be another device applicable to blood pressure detection or another scenario.

Alternatively, the electronic device may be another device that needs to control fluidity of fluid, for example, may be a medical device that monitors blood pressure or implements another function.

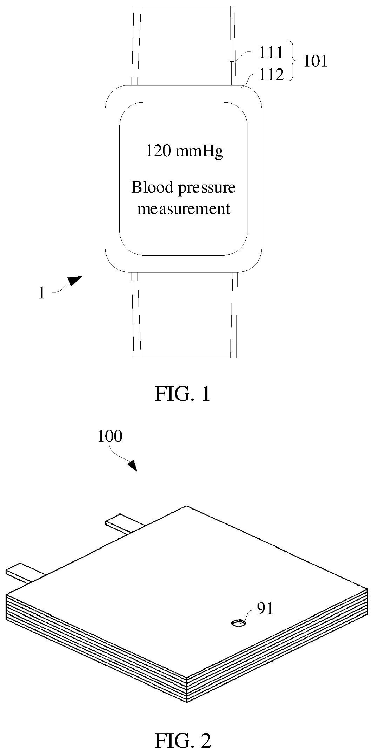

FIG. 1 is a schematic diagram of a structure of an electronic device according to an embodiment of this application.

In this embodiment of this application, as shown in FIG. 1, an example in which the electronic device 1 is a watch and a micro fluid control apparatus included in the electronic device 1 is a micro air pump is used to describe an application scenario of the micro fluid control apparatus.

For example, the electronic device 1 may include a housing 101. Refer to FIG. 1. Specifically, the housing 101 may include a watch face 112 and a wristband 111. The watch face 112 is disposed on the wristband 111. The electronic device 1 may further include an air bag (not shown in the figure) and the micro air pump (not shown in the figure). The micro air pump may be disposed in the watch face 112, and the air bag may be disposed in the wristband 111. Alternatively, in some other examples, the micro air pump and the air bag may be respectively disposed in the wristband 111 and the watch face 112, or both the micro air pump and the air bag may be disposed in the wristband 111 or the watch face 112.

For example, the micro air pump may be disposed in the watch face 112, and the air bag may be disposed in the wristband 111. The wristband 111 is in contact with a body surface. The micro air pump can pump air. The air bag may be inflated by using the micro air pump. In processes of inflating and deflating the air bag, a change trend of an amplitude of an oscillation wave of an arterial vessel in a process in which the air bag applies pressure to the body surface and performs blocking is measured. Systolic blood pressure (Systolic Blood Pressure, SBP) and diastolic blood pressure (Diastolic Blood Pressure, DBP) may be calculated based on a pressure change in the air bag measured by a barometer, to detect blood pressure of a user. In this way, a function of medical-level blood pressure detection of the electronic device 1 is implemented.

The following describes a specific structure of the micro fluid control apparatus by using an example in which the micro fluid apparatus is a micro air pump.

FIG. 2 is a schematic diagram of a structure of a micro fluid control apparatus according to an embodiment of this application.

Usually, to reduce a volume of the micro fluid control apparatus 100, the micro fluid apparatus is often in a stacking structure. For example, as shown in FIG. 2, the micro fluid control apparatus 100 is a stacking micro air pump. An air vent hole 91 is provided on the micro air pump. The air vent hole 91 may be configured to communicate with an air bag. The micro air pump can control a flow rate and pressure of air, and pump the air out from the air vent hole 91, to pump out the air in the air bag.

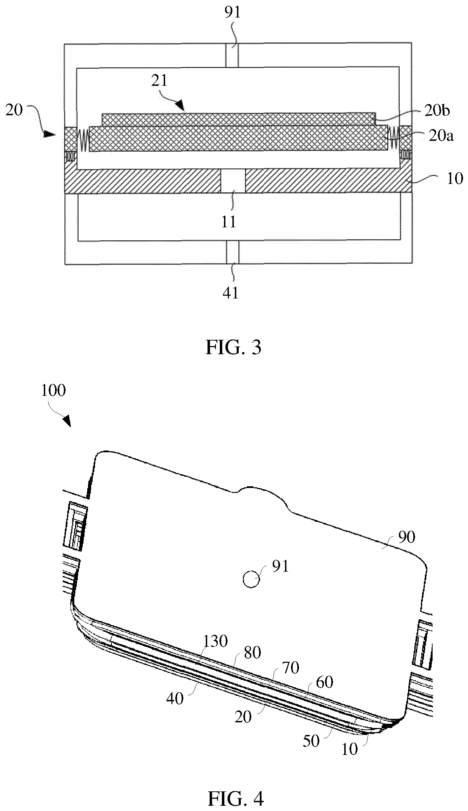

FIG. 3 is a schematic diagram of a partial cross-sectional structure of a micro fluid control apparatus according to an embodiment of this application.

Refer to FIG. 3. The micro fluid control apparatus 100 may include an oscillator layer 20 and a resonance layer 10 that are stacked. A vibration portion 21 of the oscillator layer 20 can vibrate. For example, the oscillator layer 20 may include a metal base 20a and a piezoelectric ceramic 20b disposed on the metal base 20a. The metal base 20a can be driven to vibrate together through a piezoelectric characteristic of the piezoelectric ceramic 20b.

An air intake hole 41 is further provided on the micro fluid control apparatus 100. An air intake opening 11 that communicates with the air intake hole 41 is provided on the resonance layer 10. There is a spacing between the oscillator layer 20 and the resonance layer 10. Specifically, there is the spacing between the vibration portion 21 of the oscillator layer 20 and the resonance layer 10, so that the vibration portion 21 can vibrate up and down. In this way, an inhalation chamber 120 is formed between the oscillator layer 20 and the resonance layer 10.

When the vibration portion 21 of the oscillator layer 20 vibrates, a size of space of the inhalation chamber 120 changes, and a flow rate and pressure of an air flow in the inhalation chamber 120 are changed. In this way, the air flow can be pumped into the inhalation chamber 120 from the air intake hole 41 and the air intake opening 11, and pumped out of the inhalation chamber 120 through the air vent hole 91, to pump air.

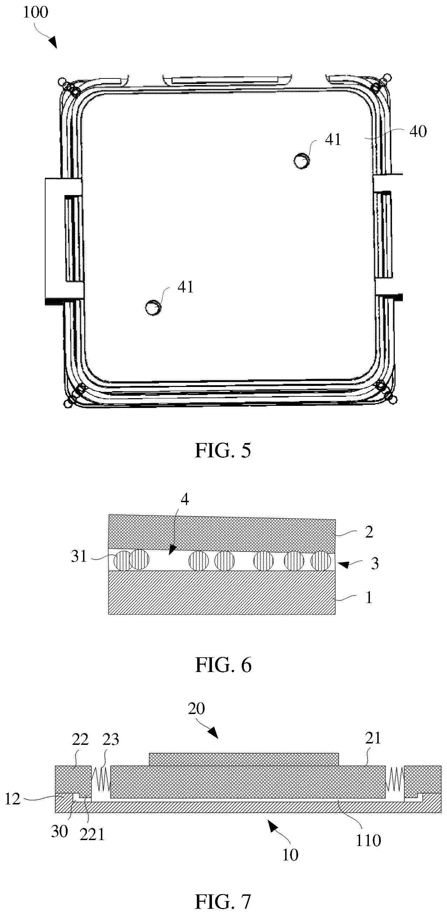

FIG. 4 is a schematic diagram of a structure of another micro fluid control apparatus from a view angle according to an embodiment of this application. FIG. 5 is a schematic diagram of a structure of another micro fluid control apparatus from another view angle according to an embodiment of this application.

Specifically, an example in which the micro air pump is a micro piezoelectric pump is used. Refer to FIG. 4. From bottom to top, the micro fluid control apparatus 100 may include an air intake layer 40, an air intake channel 50, a resonance layer 10, an oscillator layer 20, an oscillator electrode 130, a front chamber structure layer 60, an air leakage diaphragm 70, an air vent channel 80, and an air vent layer 90 that are stacked. The oscillator electrode 130 is configured to drive a vibration portion of the oscillator layer 20 to bend and vibrate up and down. A spacing (not shown in the figure) needs to be reserved between the resonance layer 10 and the oscillator layer 20, to ensure that the vibration portion has required vibration space. In this way, an inhalation chamber is formed between the resonance layer 10 and the oscillator layer 20. Refer to FIG. 5. An air intake hole 41 is provided on the air intake layer 40. The air intake hole 41 communicates with an air intake opening 11 on the resonance layer 10 through the air intake channel 50. An air flow may enter the inhalation chamber through the air intake hole 41, the air intake channel 50, and the air intake opening 11.

The vibration portion of the oscillator layer 20 vibrates up and down, so that sizes of the spacing between the oscillator layer 20 and the resonance layer 10 and space of the inhalation chamber change. In this way, the air flow is pumped out of the inhalation chamber. An air discharge opening is provided on the oscillator layer 20. The air discharge opening communicates with the front chamber structure layer 60. The front chamber structure layer 60 communicates with the air leakage diaphragm 70. The air leakage diaphragm 70 communicates with the air vent layer 90 through the air vent channel 80. An air vent hole 91 may be provided on the air vent layer 90. An air flow pumped out by the resonance layer 10 through the air discharge opening flows to the air vent layer 90 through the front chamber structure layer 60, the air leakage diaphragm 70, and the air vent channel 80, and is discharged through the air vent hole 91 on the air vent layer 90.

During blood pressure detection, an air flow enters from the air intake layer 40 and passes through the air intake channel 50. When the vibration portion of the oscillator layer 20 bends and vibrates up and down at a high frequency, the air flow is pumped into the front chamber structure layer 60, flows out through the air leakage diaphragm 70, the air vent channel 80, and the air vent layer 90 in sequence, and flows into an air bag that communicates with the micro fluid control apparatus 100, to inflate the air bag.

It can be seen that, the spacing between the resonance layer 10 and the oscillator layer 20 has great impact on performance of the micro fluid control apparatus 100. If a height of the spacing is not appropriately controlled (excessively large, excessively small, or uneven), control performance of the micro fluid control apparatus 100 on a flow of fluid is directly affected, and performance of the micro fluid control apparatus is poor. A micro air pump (such as a micro piezoelectric pump) is used as an example. When the height of the spacing is large, a flow rate and back pressure of the air flow decrease. Consequently, air pumping performance of the micro air pump is affected. However, when the height of the spacing is small, the resonance layer 10 and the oscillator layer 20 are likely to collide in a process of vibrating up and down. This causes an abnormal sound, may also cause damage to the resonance layer 10 and the oscillator layer 20, and reduces reliability. When the height of the spacing is not consistent, the flow rate and pressure of the air flow are inconsistent. The air pumping performance of the micro air pump is also affected.

FIG. 6 is a schematic diagram of a cross-sectional structure of existing assembly of a resonance layer and an oscillator layer.

To ensure that there is a spacing between a resonance layer and an oscillator layer, in a conventional technology, microbead glue is used as a support for forming the spacing. Specifically, as shown in FIG. 6, a glue layer 3 having ring-shaped microbeads exists between a resonance layer 1 and an oscillator layer 2. The microbead glue layer is a glue layer formed on the resonance layer 1 in a dispensing manner after microbeads 31 are mixed into glue. The resonance layer is fixedly connected to the oscillator layer by using the glue layer. The microbeads 31 mixed into the glue layer can provide a support function, so that a spacing 4 is reserved between the resonance layer 1 and the oscillator layer 2. However, a height of the spacing 4 depends on a height of the formed microbead glue layer 3.

However, because sizes of the microbeads 31 are small, for ease of assembly, the microbeads 31 are first mixed into the glue, and then the glue mixed with the microbeads 31 is dispensed on the resonance layer 1. Distribution of the microbeads 31 in the glue is uncontrollable. Consequently, the height of the formed spacing 4 is uncontrollable, and the height of the spacing 4 is difficult to remain consistent.

For example, it is difficult to evenly distribute the microbeads 31 in the glue one by one, and agglomeration is likely to occur. In this way, the oscillator layer is supported by using the microbead glue, to form the spacing 4, and the height of the spacing 4 is difficult to remain consistent. It is also difficult to control distribution positions of the microbeads 31 in the glue. After dispensing is performed, it is also difficult to control the distribution positions of the microbeads 31. Consequently, the height of the spacing 4 is inconsistent. Poor consistency of the height of the spacing 4 can reduce consistency of air flow and back pressure of a micro air pump. Consequently, performance of the micro air pump is reduced.

In addition, the height of the spacing 4 is determined by a height of the microbead glue layer, and a quantity of glue is difficult to control. In a process in which the glue mixed with the microbeads is used to form the microbead glue layer in a dispensing manner, a problem that the glue overflows toward a vibration layer is likely to occur. Consequently, a problem of blocking a valve by the glue occurs, and vibration of the vibration layer is suppressed.

Based on this, a micro fluid control apparatus provided in embodiments of this application can accurately control a height of a spacing between a resonance layer and an oscillator layer, to improve performance of the micro fluid control apparatus.

The following describes in detail assembly of the resonance layer and the oscillator layer in the micro fluid control apparatus provided in embodiments of this application with reference to the accompanying drawings.

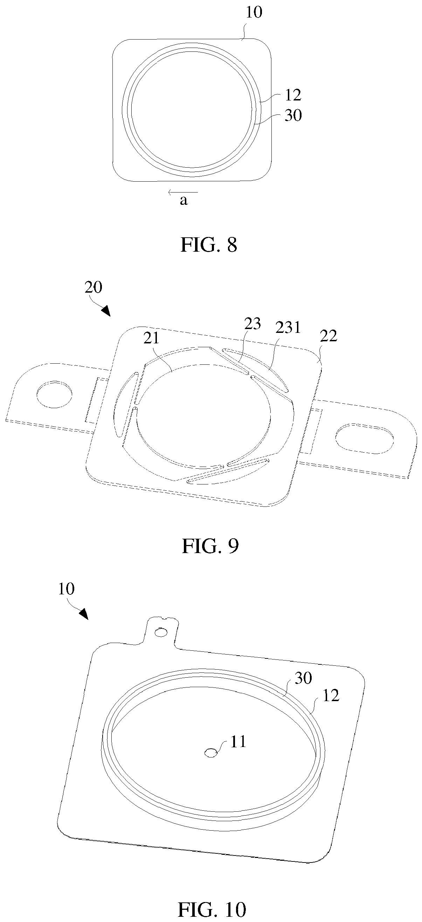

FIG. 7 is a schematic diagram of a cross-sectional structure of assembly of a resonance layer and an oscillator layer according to an embodiment of this application. FIG. 8 is a schematic top view of assembly of a resonance layer and a bonding layer according to an embodiment of this application.

Refer to FIG. 7. The oscillator layer 20 and the resonance layer 10 are stacked. The oscillator layer 20 may include a vibration portion 21, a fastening portion 22, and a connection portion 23. The fastening portion 22 encloses an outer periphery of the vibration portion 21. The vibration portion 21 is connected to the fastening portion 22 by using the connection portion 23. The vibration portion 21 can bend and vibrate up and down (for example, in a micro piezoelectric pump, the vibration portion 21 can vibrate under an action of an oscillator electrode). When the resonance layer 10 is connected to the oscillator layer 20, the fastening portion 22 is fixedly connected to the resonance layer 10.

A bonding layer 30 is provided between the oscillator layer 20 and the resonance layer 10. Specifically, the bonding layer 30 is located between the fastening portion 22 and the resonance layer 10. The fastening portion 22 is fixedly connected to the resonance layer 10 by using the bonding layer 30, so that the oscillator layer 20 and the resonance layer 10 are assembled and fastened. The bonding layer 30 may be formed between the resonance layer 10 and the oscillator layer 20 in a manner of dispensing colloid.

A support structure 12 is further provided between the oscillator layer 20 and the resonance layer 10. Specifically, the support structure 12 is located between the resonance layer 10 and the fastening portion 22, so that a spacing 110 is formed between the oscillator layer 20 and the resonance layer 10, and the reserved spacing 110 can meet space required by the vibration portion 21 to vibrate. In addition, the support structure 12, the oscillator layer 20, and the resonance layer 10 can form, in an enclosing manner, a chamber that can be configured to accommodate fluid and allow the fluid to pass through. An inlet (not shown in the figure) for the fluid to enter the chamber may be provided on the resonance layer 10. A through opening (not shown in the figure) for the fluid to flow out of the chamber may be provided on the oscillator layer 20.

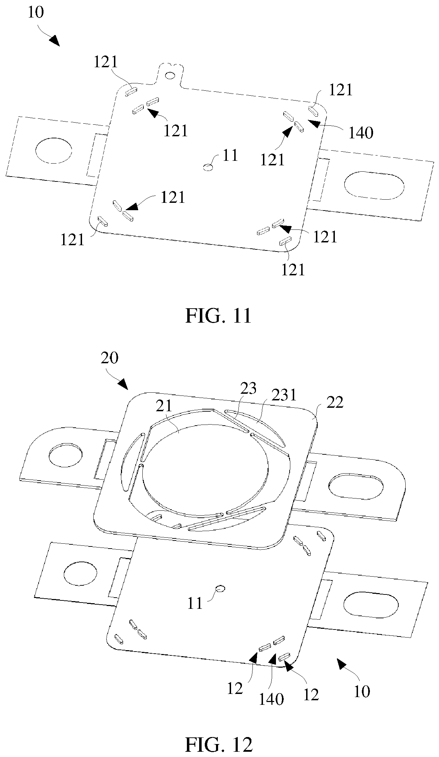

When the vibration portion 21 vibrates at a high frequency relative to the resonance layer 10, sizes of the spacing 110 between the oscillator layer 20 and the resonance layer 10 and the formed chamber change. In this way, a flow rate and pressure of the fluid located in the chamber are changed, and the fluid can be pumped into the chamber from the inlet and pumped out of the chamber from the through opening, to control a flow of the fluid. For example, the micro fluid control apparatus 100 is a micro piezoelectric pump. When the vibration portion 21 of the oscillator layer 20 vibrates at a high frequency, an air flow can enter an inhalation chamber 120 from an air intake opening 11 and be pumped out from an air discharge opening 231 (refer to FIG. 12), to pump air.

The connection portion 23 may be an elastic part, for example, may be a spring part, to ensure that the vibration portion 21 has good vibration effect. One end of the connection portion 23 may be connected to the vibration portion 21, and the other end of the connection portion 23 may be connected to the fastening portion 22. A plurality of connection portions 23 may be disposed between the vibration portion 21 and the fastening portion 22. There may be a spacing between two adjacent connection portions 23, to form the through opening (for example, the air discharge opening) on the oscillator layer 20.

Alternatively, the connection portion 23 may be another mechanical part having a deformation margin. For example, the connection portion 23 may be a metal sheet. Two ends of the connection portion 23 may be located on the fastening portion 22 (refer to FIG. 12), and the vibration portion 21 is connected to the connection portion 23. This helps improve strength of the connection portion 23 and ensure vibration stability of the vibration portion 21.

The air discharge opening 231 may be located on the connection portion 23, or there may be spacings 110 between two adjacent connection portions 23 and between the connection portion 23 and the fastening portion 22, to form the air discharge opening 231.

Still refer to FIG. 7. The bonding layer 30 may surround the outer periphery of the vibration portion 21. For example, the vibration portion 21 may be circular. With reference to FIG. 8, the bonding layer 30 is a closed ring that surrounds the outer periphery of the vibration portion 21. In this way, sealing performance of the formed chamber (for example, the inhalation chamber 120) in a circumferential direction can be effectively improved. This helps ensure control of a flow of the fluid.

Still refer to FIG. 8. The support structure 12 is located in a radial direction of the bonding layer 30. The radial direction is a direction perpendicular to a center line of the bonding layer 30. For example, the radial direction may be a direction a in FIG. 8. In other words, the support structure 12 may be located on a circumferential inner side (a side close to a center of the ring-shaped bonding layer 30) of the bonding layer 30. Alternatively, the support structure 12 may be located on a circumferential outer side (a side opposite to an inner side) of the bonding layer 30. Alternatively, there may be a plurality of support structures 12. Some support structures 12 may be located on a circumferential inner side of the bonding layer 30, and some support structures 12 may be located on a circumferential outer side of the bonding layer 30.

In other words, the support structure 12 and the bonding layer 30 are located in a same plane between the oscillator layer 20 and the resonance layer 10. Two ends of the bonding layer 30 are respectively connected to the oscillator layer 20 and the resonance layer 10, so that the resonance layer 10 is fixedly connected to the oscillator layer 20. The bonding layer 30 provides a good connection function. The bonding layer 30 does not exist between the support structure 12 and the oscillator layer 20 or between the support structure 12 and the resonance layer 10. Two ends of the support structure 12 are also connected to the oscillator layer 20 and the resonance layer 10 respectively. The support structure 12 provides a good support function. The spacing 110 is reserved between the oscillator layer 20 and the resonance layer 10 by using a support height of the support structure 12. In other words, a height of the spacing 110 depends on a height of the support structure 12.

The height of the support structure 12, a disposing position of the support structure 12, and the like are easily controlled. For example, a preparation process condition or the like of the support structure 12 can be adjusted, to adjust the height and the position of the support structure 12. In this way, the height of the spacing 110 between the oscillator layer 20 and the resonance layer 10 is accurately controlled, and the height of the spacing 110 can be accurately remained at a required specified value. This can avoid a problem that the flow rate and back pressure of the fluid are excessively small due to an excessively large height of the spacing 110, and ensure performance of the micro fluid control apparatus 100. In addition, this can avoid a problem of collision of the resonance layer and the oscillator layer during vibration due to an excessively small height of the spacing 110, reduce or avoid problems of an abnormal sound of impact and poor reliability due to the impact, and improve a yield rate of the micro fluid control apparatus 100 and user experience.

In addition, because the height of the support structure 12 is easy to control, consistency of the height of the spacing 110 between the resonance layer 10 and the oscillator layer 20 can be ensured. This improves control performance on the fluid and improves performance of the micro fluid control apparatus 100. For example, the micro fluid control apparatus 100 is a micro piezoelectric pump. The height of the spacing 110 between the oscillator layer 20 and the resonance layer 10 is consistent. This helps improve consistency of an air flow and back pressure of the micro piezoelectric pump, and improve air pumping performance of the micro piezoelectric pump.

In addition, because the height and the disposing position of the support structure 12 are easily controlled, the position and the height of the support structure 12 can be adjusted based on a specific vibration situation of the micro fluid control apparatus 100, to adjust and optimize the spacing 110 between the oscillator layer 20 and the resonance layer 10. This further helps improve performance of the micro fluid control apparatus 100.

In addition, the bonding layer 30 only connects the oscillator layer 20 and the resonance layer 10. The height of the support structure determines the height of the spacing 110. This facilitates control of a quantity of the colloid used when the bonding layer 30 is formed, and reduces or avoids a problem of suppressing vibration of the oscillator layer due to overflow of the colloid.

It should be noted that, the support structure 12 may be formed on or fixedly disposed on the oscillator layer 20. For example, the support structure 12 may be disposed, in a protruding manner, on a surface that is of the fastening portion 22 of the oscillator layer 20 and that faces the resonance layer 10, and the resonance layer 10 abuts against the support structure 12.

Alternatively, the support structure 12 may be formed or fixedly disposed on the resonance layer 10. For example, the support structure 12 may be disposed, in a protruding manner, on a surface that is of the resonance layer 10 and that faces the oscillator layer 20, the support structure 12 is connected to the resonance layer 10, and the fastening portion 22 of the oscillator layer 20 abuts against the support structure 12. In this way, the spacing 110 is formed between the resonance layer 10 and the oscillator layer 20 by using the support structure 12. Compared with disposing the support structure 12 on the oscillator layer 20 whose original structure is complex, the support structure 12 is disposed on the resonance layer 10, so that difficulty in molding the oscillator layer can be reduced. This helps implement mass production. In embodiments of this application, an example in which the support structure 12 is disposed on the resonance layer 10 is used for description.

When the oscillator layer 20 and the resonance layer 10 are assembled, for example, the support structure 12 may be first formed on the resonance layer 10. Then, the bonding layer 30 is formed on the resonance layer 10 in a dispensing manner. The support structure 12 is located in the radial direction of the bonding layer 30. Then, the oscillator layer 20 is disposed on the support structure 12 and the bonding layer 30, so that the oscillator layer 20 is fixedly connected to the resonance layer 10 by using the bonding layer 30.

FIG. 9 is a schematic diagram of a structure of an oscillator layer according to an embodiment of this application.

For example, the micro fluid control apparatus 100 is a micro piezoelectric pump. Refer to FIG. 9. The oscillator layer 20 includes a vibration portion 21, a fastening portion 22, and a connection portion 23. The connection portion 23 may be a metal dome. The vibration portion 21 may be circular. The fastening portion 22 may enclose an outer periphery of the vibration portion 21. An air discharge opening 231 is formed between the connection portion 23 and the fastening portion 22. The vibration portion 21 may bend and vibrate up and down under an action of an oscillator electrode.

FIG. 10 is a schematic top view of a structure of a resonance layer according to an embodiment of this application.

Refer to FIG. 10. An air intake opening 11 may be provided at a middle part of the resonance layer 10. A support structure 12 is further disposed on the resonance layer 10. A bonding layer 30 may be formed on the resonance layer 10 in a dispensing manner. The support structure 12 may be located in a radial direction of the bonding layer 30. For example, the bonding layer 30 is a ring. The support structure 12 may be a closed ring. The support structure 12 is located on a circumferential outer side of the bonding layer 30. The support structure 12 and the bonding layer 30 may be two rings disposed in a concentric manner.

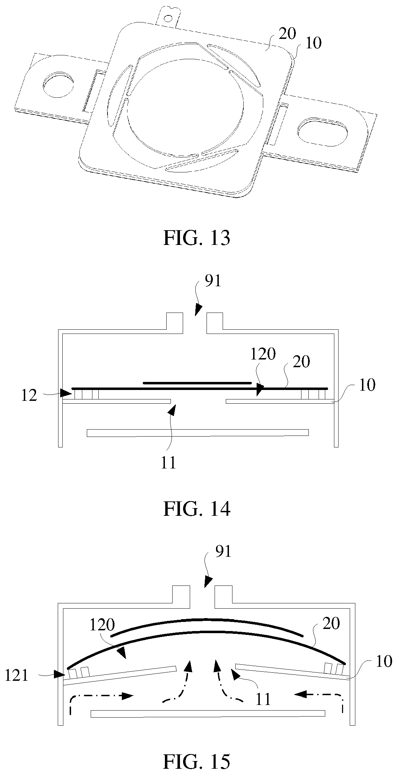

FIG. 11 is a schematic diagram of a structure of another resonance layer according to an embodiment of this application. FIG. 12 is a schematic diagram of splitting of an oscillator layer and a resonance layer according to an embodiment of this application. FIG. 13 is a schematic diagram of a structure obtained by assembling an oscillator layer and a resonance layer according to an embodiment of this application.

Alternatively, the support structure 12 may be an unclosed ring. Refer to FIG. 11, the support structure 12 includes a plurality of support parts 121. Specifically, an example in which each support structure 12 includes four support parts 121 is used. The four support parts 121 are spaced from each other. For example, the four support parts 121 located at four corners of the resonance layer 10 form the unclosed ring-shaped support structure 12. The ring-shaped support structure 12 is located in a radial direction of the bonding layer. The ring-shaped support structure 12 and the bonding layer may also form a concentric ring. As shown in FIG. 11, in this way, two support structures 12 are formed on the resonance layer 10. One support structure may be located on an inner side (a side adjacent to the air intake opening 11) of the other support structure. The bonding layer may be located in a gap 140 between the two support structures.

Each support part 121 may be a single support structure body, or each support part 121 may include two or more sub-support structure bodies (refer to the support structure located on an inner side in FIG. 11, and each support part includes two sub-support bodies).

With reference to FIG. 12 and FIG. 13, the oscillator layer 20 is stacked on the resonance layer 10, so that the fastening portion 22 of the oscillator layer 20 is connected to the resonance layer 10 by using the bonding layer. In this way, the oscillator layer 20 and the resonance layer 10 are assembled and fastened. In addition, the support structure 12 is provided between the fastening portion 22 and the resonance layer 10, so that the spacing is formed between the oscillator layer 20 and the resonance layer 10 by using the support structure 12.

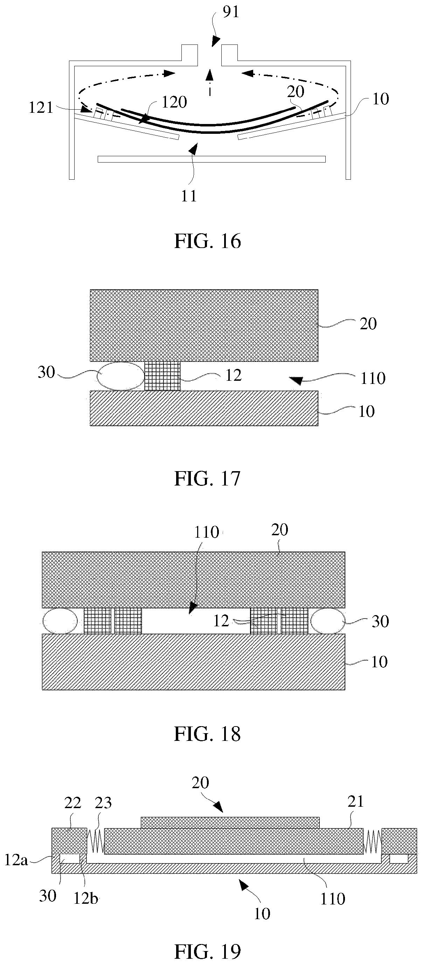

FIG. 14 is a schematic diagram of fitting of a resonance layer and an oscillator layer obtained when a micro fluid control apparatus is in a non-working state according to an embodiment of this application. FIG. 15 is a schematic diagram of fitting of a resonance layer and an oscillator layer obtained when a micro fluid control apparatus is in a first working state according to an embodiment of this application. FIG. 16 is a schematic diagram of fitting of a resonance layer and an oscillator layer obtained when a micro fluid control apparatus is in a second working state according to an embodiment of this application.

Refer to FIG. 14. The spacing is formed between the oscillator layer 20 and the resonance layer 10 by using the support structure 12. In this way, the support structure 12, the oscillator layer 20, and the resonance layer 10 can form the inhalation chamber 120 in an enclosing manner. The air intake opening 11 is provided at the middle part of the resonance layer 10. The air intake opening 11 communicates with the inhalation chamber 120. The air discharge opening (not shown in the figure) that communicates with the inhalation chamber 120 is provided between the fastening portion 22 and the vibration portion 21 of the oscillator layer 20.

When blood pressure needs to be detected, that is, the micro fluid control apparatus 100 is in a working state, the vibration portion of the oscillator layer 20 bends and vibrates up and down. Bending and vibrating upward is a first working state, and bending and vibrating downward is a second working state.

Refer to FIG. 15. When the micro fluid control apparatus 100 is in the first working state, the oscillator layer 20 bends and vibrates upward. In this case, an accommodating area of the inhalation chamber 120 becomes large. An air flow enters the inhalation chamber 120 from the air intake opening 11 located at the middle part of the resonance layer 10.

Refer to FIG. 16. When the micro fluid control apparatus 100 is in the second working state, the oscillator layer 20 bends and vibrates downward. In this case, the accommodating area of the inhalation chamber 120 becomes small. The air flow is discharged from the air discharge opening located on the outer periphery of the fastening portion 22. The resonance layer 10 and the oscillator layer 20 repeatedly bend and vibrate up and down at a high frequency, so that the air flow can be pumped from the air intake opening 11 to the air discharge opening, and then pumped out from the air vent hole 91, to pump air and inflate an air bag.

A height range of the support structure 12 may be 5 µm to 12 µm. In this way, the height of the spacing 110 between the oscillator layer 20 and the resonance layer 10 is 5 µm to 12 µm. The height of the spacing 110 within this range is not too large to cause an excessively small flow rate and pressure of the fluid in the chamber, and is not too small to cause collision of the oscillator layer 20 and the resonance layer 10 during vibration and problems of an abnormal sound of impact and poor reliability.

In embodiments of this application, the support structure 12 is located in a radial direction of the bonding layer 30. Specifically, the support structure 12 may be located on a circumferential outer side of the bonding layer 30 (refer to FIG. 7 and FIG. 8). The resonance layer 10 and the support structure 12 located on the resonance layer 10 may form glue accommodating space. The bonding layer 30 may be disposed in the glue accommodating space. In this way, the resonance layer 10, the oscillator layer 20, and the support structure 12 are connected by using the bonding layer 30. This helps improve stability of the micro fluid control apparatus 100.

Specifically, refer to FIG. 7. A protrusion portion 221 may be formed on a surface that is of the fastening portion 22 of the oscillator layer 20 and that faces the resonance layer 10. The protrusion portion 221, the resonance layer 10, and the support structure 12 may jointly form accommodating space in an enclosing manner. The bonding layer 30 may be located in the accommodating space. In this way, the bonding layer 30 can be conveniently disposed, and contact areas between the resonance layer 10 and the oscillator layer 20, and the bonding layer 30 can also be increased. This enhances connection fastness between the resonance layer 10 and the oscillator layer 20 and improves sealing performance of the chamber.

It should be noted that, the protrusion portion 221 protrudes toward the resonance layer 10, there is a gap between the protrusion portion 221 and the resonance layer 10, and the bonding layer 30 is filled in the gap. In other words, the protrusion portion 221 does not protrude to abut against the resonance layer 10. In this way, impact of a height of the protrusion portion 221 on the spacing 110 caused because the protrusion portion 221 abuts against the resonance layer 10 can be avoided. This can reduce machining precision of the protrusion portion 221, reduce difficulty in fitting and assembling the resonance layer 10 and the oscillator layer 20, and reduce difficulty in machining the entire micro fluid control apparatus.

To reduce or avoid impact on vibration of the vibration portion 21 caused by glue overflow occurred in a process of forming the bonding layer 30 in a dispensing manner, a glue overflow groove (not shown in the figure) may be provided on the resonance layer 10. For example, the glue overflow groove may be provided at a position that is on the resonance layer 10 and that is opposite to the protrusion portion 221.

FIG. 17 is a schematic diagram of a cross-sectional structure obtained by fitting another oscillator layer with another resonance layer according to an embodiment of this application. FIG. 18 is a schematic diagram of a partial cross-sectional structure obtained by fitting still another oscillator layer with still another resonance layer according to an embodiment of this application.

Alternatively, refer to FIG. 17. The support structure 12 may be located on the circumferential inner side of the bonding layer 30. The support structure 12 may block and limit the bonding layer 30. This reduces or avoids problems such as glue overflow caused by diffusion of colloid toward the vibration portion 21 of the oscillator layer 20 in a process of forming the bonding layer 30 in a dispensing manner, and further ensures vibration performance of the vibration portion 21.

In embodiments of this application, there may be two or more support structures 12 between the resonance layer 10 and the oscillator layer 20. The two or more support structures 12 may be located on the circumferential inner side of the bonding layer 30. For example, refer to FIG. 18. An example in which the resonance layer 10 is provided with two support structures 12 is used. The two support structures 12 may be located on the inner side of the bonding layer 30.

FIG. 19 is a schematic diagram of a cross-sectional structure obtained by fitting yet another oscillator layer with yet another resonance layer according to an embodiment of this application.

Alternatively, some support structures may be located on the circumferential inner side of the bonding layer 30, and some support structures may be located on the circumferential outer side of the bonding layer 30.

For example, there may be the gap 140 (refer to FIG. 11) between the two support structures 12, to form an accommodating groove. Refer to FIG. 19. An example in which two support structures are provided between the resonance layer 10 and the oscillator layer 20, and are respectively a support structure 12a and a support structure 12b is used. The support structure 12a and the support structure 12b are spaced from each other, so that the accommodating groove is formed between the support structure 12a and the support structure 12b. The bonding layer 30 may be located in the accommodating groove.

In other words, in the two support structures 12, one support structure (for example, the support structure 12b) is located on the circumferential inner side of the bonding layer 30, and the other support structure 12 (the support structure 12a) is located on the circumferential outer side of the bonding layer 30. The accommodating groove can limit the bonding layer 30. This can facilitate dispensing of colloid, to form the bonding layer 30, improve stability of the colloid, and effectively reduce overflow of the colloid. In addition, this also helps increase contact areas between the support structure 12 and the oscillator layer 20, and the bonding layer 30, and ensure bonding fastness between the resonance layer 10 and the oscillator layer 20.

In embodiments of this application, the support structure 12 may be a single mechanical part. For example, the support structure 12 may be a ring-shaped structure disposed on the resonance layer 10 in a protruding manner. The support structure 12 may be a closed ring. Certainly, in some other examples, the support structure 12 may alternatively be an unclosed ring.

The support structure 12 is in a closed ring shape, so that the oscillator layer 20 can be evenly supported, and the consistency of the height of the spacing 110 can be further ensured. In addition, a molding manner is simple and easy to operate.

Specifically, for example, the support structure 12 may be a closed and circular ring. For example, the closed and circular ring-shaped support structure 12 that is connected head to tail is formed on the resonance layer 10.

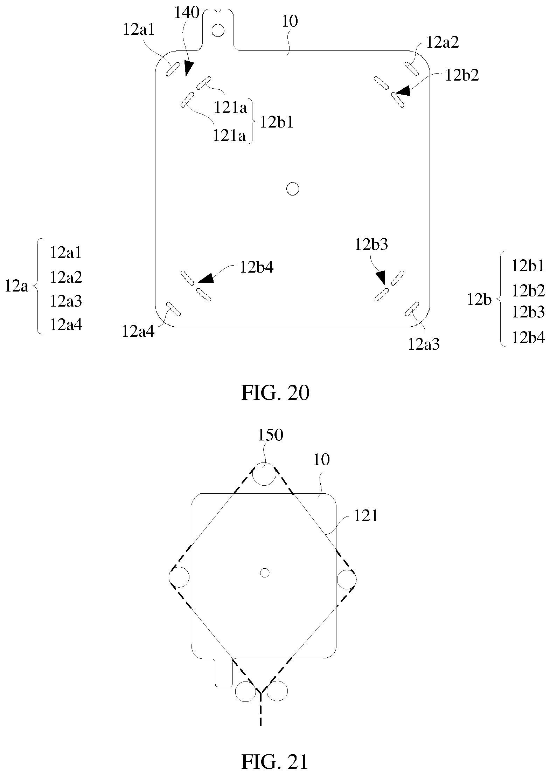

FIG. 20 is a schematic diagram of a support structure on a resonance layer according to an embodiment of this application.

Alternatively, in some examples, the support structure may include a plurality of support parts 121. Refer to FIG. 20. Two support structures are disposed on the resonance layer 10, and are respectively a support structure 12a and a support structure 12b. An example in which each support structure includes four support parts 121 is used. The support structure 12a include a support part 12a1, a support part 12a2, a support part 12a3, and a support part 12a4. The support part 12a1, the support part 12a2, the support part 12a3, and the support part 12a4 are spaced from each other and evenly disposed in an enclosing manner, so that the support structure 12a is of a ring-shaped structure.

The support structure 12b located on an inner side of the support structure 12a may also include four support parts 121: a support part 12b 1, a support part 12b2, a support part 12b3, and a support part 12b4. The support part 12b 1, the support part 12b3, the support part 12b2, and the support part 12b4 are spaced from each other and evenly disposed in an enclosing manner, so that the support structure 12b is of a ring-shaped structure.

The support parts 121 are evenly distributed to form the ring-shaped support structure. This reduces costs and ensures evenness of disposition of the support parts 121 on the resonance layer 10. In this way, consistency of the height of the spacing between the oscillator layer 20 and the resonance layer 10 is improved, and performance of the micro fluid control apparatus 100 is improved.

Specifically, the support structure 12 may be a circular ring. For example, the support part 12bl, the support part 12b2, the support part 12b3, and the support part 12b4 are evenly distributed and formed the circular ring in an enclosing manner.

In embodiments of this application, the support part 121 may include at least two sub-support bodies 121a. An example in which each support part 121 in the support structure 12b in FIG. 20 includes two sub-support bodies 121a is used. For example, the support part 12b1 includes two sub-support bodies 121a, and two adjacent sub-support bodies 121a are spaced from each other. In this way, materials required for molding the support parts 121 can be reduced. This helps further reduce the costs and facilitates a weight reduction design of the micro fluid control apparatus 100.

It should be noted that the resonance layer 10 is provided with at least two support structures 12, and each support structure 12 includes at least two support parts 121. Support parts 121 in all support structures 12 include at least two sub-support bodies 121a, or support parts 121 in some support structures 12 include at least two sub-support bodies 121a. Refer to FIG. 20. The resonance layer 10 is provided with two support structures 12, and only the support parts 121 in the support structure 12b include two sub-support bodies 121a.

In embodiments of this application, the support structure 12 may be molded in a plurality of manners. An example in which the support structure 12 is fastened to the resonance layer 10 is used. Specifically, in a possible implementation, the support structure 12 and the resonance layer 10 may be integrally molded. Because the height of the support structure 12 is usually required to be at a micrometer level, using an integrally molding manner can facilitate molding and assembling of the support structure 12, reduce machining and assembling difficulty, and help improve control precision of the height of the spacing 110.

For example, a protrusion (refer to FIG. 7) is provided on a surface that is of the resonance layer 10 and that faces the oscillator layer 20. The protrusion forms the support structure 12, so that the spacing 110 is reserved between the oscillator layer 20 and the resonance layer 10. Specifically, the support structure 12 may be integrally formed on the resonance layer 10 in a process of forming the resonance layer 10. For example, when the resonance layer 10 is formed, the protrusion may be integrally formed, through a computer numerical control (Computerized Numerical Control, CNC for short) machining process, on a surface that is of the resonance layer 10 and that faces the oscillator layer 20. Alternatively, after the resonance layer 10 is formed, the protrusion is formed on the resonance layer 10 in a manner such as etching, to form the support structure 12.

The support structure 12 formed through a CNC machining process, etching, or the like has high precision, so that the height of the spacing 110 between the oscillator layer 20 and the resonance layer 10 can be further accurately controlled.

Alternatively, in another possible implementation, the support structure 12 and the resonance layer 10 may be separately molded. The molded support structure 12 may be disposed on a surface that is of the resonance layer 10 and that faces the oscillator layer 20. This improves disposition flexibility of the support structure 12 and enriches application scenarios of the micro fluid control apparatus 100.

Specifically, for example, the support structure 12 may be a plating layer. For example, in a possible implementation, the support structure 12 may include a plating layer. For example, the plating layer may be a metal plating layer, and the support structure 12 may be the metal plating layer formed on the resonance layer 10.

A molding manner of the metal plating layer may be a sputtering plating layer, a vacuum plating layer, or the like, or may be another molding manner. Specifically, for example, a plurality of metal plating layers may be formed on the resonance layer 10 in a sputtering plating layer manner. The support structure 12 includes the plurality of metal plating layers, to support the oscillator layer 20. The molding manner is simple and fast, and has high feasibility.

FIG. 21 is a schematic diagram of molding of another support structure according to an embodiment of this application.

In another possible implementation, the support structure 12 may include a metal wire. For example, a plurality of metal wires may be laid on the resonance layer 10, and the support structure 12 includes the plurality of metal wires, to support the oscillator layer 20.

The metal wire may be a steel wire, or may be another type of metal wire. Inner and outer diameters of the metal wire have high controllability. This helps further improve the consistency of the height of the spacing 110. For example, the metal wire is used as the support structure 12, so that a height difference of the spacing 110 between the oscillator layer 20 and the resonance layer 10 is less than or equal to 1 µm.

Based on a height requirement of the support structure 12, there may be one layer of metal wires. Alternatively, there may be at least two layers of metal wires. An example in which the support structure 12 includes the support parts 121 is used. One support part 121 may include at least two layers of metal wires, and the at least two layers of metal wires are stacked, to form the support part 121. In other words, the metal wires are stacked on a surface that is of the resonance layer 10 and that faces the oscillator layer 20, to form the support part 121.

Refer to FIG. 21. A columnar fastener 150 may be disposed on an outer periphery of the resonance layer 10. The fastener 150 is configured to fasten the metal wires, so that the metal wires are disposed at a predetermined position, and the metal wires are stacked. After the metal wires are fastened, the redundant metal wires (as shown in dashed line parts in FIG. 21) are removed, so that the support parts 121 may be formed on the resonance layer 10, to form the support structure 12.

It should be noted that the support structure 12 is a metal wire, and the metal wire may be fastened to the resonance layer 10 in a bonding manner. Specifically, the metal wire may be fastened by using the bonding layer 30. In other words, when the resonance layer 10 is connected to the oscillator layer 20 by using bonding layer 30, the support structure 12 is fastened to the resonance layer 10. This facilitates implementation, helps reduce costs, and also helps improve bonding fastness between the resonance layer 10, the support structure 12, and the oscillator layer 20.

For example, after the metal wires are disposed with the assistance of the fastener 150, when the bonding layer 30 is formed on an outer side of the support structure 12 formed by the metal wires, the metal wires located on the resonance layer 10 may be fastened by using the bonding layer 30, and the redundant metal wires are cut off after being wound. In this way, the support structure 12 is fastened.

Certainly, in some other examples, the support structure 12 may be another type of plating layer, or may be a metal or non-metal coating, or the like. Alternatively, the support structure 12 may be another mechanical part that can provide a support function.

It should be noted that values and value ranges in embodiments of this application are approximate values, and an error within a specific range may exist due to impact of a manufacturing process. Persons skilled in the art may consider that the error is negligible.

In descriptions of embodiments of this application, it should be noted that, unless otherwise clearly specified and limited, the terms "mount", "connect to", and "connection" should be understood in a broad sense. For example, the connection may be a fixed connection, may be an indirect connection by using an intermediate medium, or may be an internal connection between two elements or an interaction relationship between two elements. Persons of ordinary skill in the art may understand specific meanings of the terms in embodiments of this application based on specific cases.

In the specification of this application, the terms "first", "second", "third", "fourth", and the like (if any) are intended to distinguish between similar objects, but do not necessarily indicate a specific order or sequence.

Finally, it should be noted that the foregoing embodiments are merely intended for describing the technical solutions of embodiments of this application other than limiting embodiments of this application. Although embodiments of this application are described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions of embodiments of this application.

## Claims

1. A micro fluid control apparatus, comprising an oscillator layer and a resonance layer that are stacked, wherein the oscillator layer comprises a vibration portion, a fastening portion, and a connection portion, the fastening portion encloses an outer periphery of the vibration portion, the vibration portion is connected to the fastening portion by using the connection portion, and a through opening for fluid to pass through is provided between the vibration portion and the fastening portion;
the micro fluid control apparatus further comprises a bonding layer and a support structure, wherein the bonding layer is located between the fastening portion and the resonance layer, the bonding layer surrounds the outer periphery of the vibration portion, and the fastening portion is connected to the resonance layer by using the bonding layer; and
the support structure is located between the resonance layer and the fastening portion, so that a spacing is formed between the oscillator layer and the resonance layer, and the support structure is located in a radial direction of the bonding layer.

2. The micro fluid control apparatus according to claim 1, wherein the support structure is connected to the resonance layer, and the oscillator layer abuts against the support structure.

3. The micro fluid control apparatus according to claim 2, wherein a protrusion is provided on a surface that is of the resonance layer and that faces the oscillator layer, to form the support structure, and the support structure and the resonance layer are integrally molded.

4. The micro fluid control apparatus according to claim 2, wherein the support structure and the resonance layer are separately molded, and the support structure is disposed on a surface that is of the resonance layer and that faces the oscillator layer.

5. The micro fluid control apparatus according to claim 4, wherein the support structure is a plating layer.

6. The micro fluid control apparatus according to claim 4, wherein the support structure is disposed on the resonance layer by using the bonding layer.

7. The micro fluid control apparatus according to claim 6, wherein the support structure is a metal wire.

8. The micro fluid control apparatus according to any one of claims 1 to 7, wherein the support structure is located on a circumferential outer side of the bonding layer.

9. The micro fluid control apparatus according to claim 8, wherein a protrusion portion is provided on a surface that is of the fastening portion and that faces the resonance layer, the protrusion portion, the resonance layer, and the support structure jointly form accommodating space, and the bonding layer is located in the accommodating space.

10. The micro fluid control apparatus according to claim 8, wherein there are at least two support structures, and at least one of the support structures is located on a circumferential inner side of the bonding layer.

11. The micro fluid control apparatus according to claim 10, wherein there is a gap between the at least two support structures, to form an accommodating groove, and the bonding layer is located in the accommodating groove.

12. The micro fluid control apparatus according to any one of claims 1 to 11, wherein the support structure is in a ring shape.

13. The micro fluid control apparatus according to claim 12, wherein the support structure is in a closed ring shape.

14. The micro fluid control apparatus according to claim 12, wherein the support structure comprises a plurality of support parts, and the plurality of support parts are spaced from each other and evenly disposed in a surrounding manner, to form the ring shape.

15. The micro fluid control apparatus according to claim 14, wherein the support part comprises at least two sub-support bodies, and two adjacent sub-support bodies are spaced from each other.

16. The micro fluid control apparatus according to claim 14, wherein a cross-sectional shape of the support part comprises at least a long strip shape, a cylindrical shape, an arc shape, and a square shape.

17. The micro fluid control apparatus according to any one of claims 1 to 16, wherein a height range of the support structure is 5 µm to 12 µm.

18. The micro fluid control apparatus according to any one of claims 1 to 17, wherein the bonding layer is not disposed between the support structure and the resonance layer or between the support structure and the oscillator layer.

19. The micro fluid control apparatus according to any one of claims 1 to 18, wherein the micro fluid control apparatus is a micro piezoelectric pump.

20. An electronic device, comprising at least a housing and the micro fluid control apparatus according to any one of claims 1 to 19, wherein the micro fluid control apparatus is disposed in the housing.

21. A micro piezoelectric pump, comprising a quasi-valve resonance diaphragm and a piezoelectric vibration diaphragm that are stacked, wherein the piezoelectric vibration diaphragm comprises a vibration portion and a plate portion that encloses an outer periphery of the vibration portion, the plate portion is connected to the vibration portion by using a connection portion, an air discharge opening is provided on the connection portion, and an air intake opening is provided at a middle part of the quasi-valve resonance diaphragm;
the micro piezoelectric pump further comprises a bonding layer located between the plate portion and the quasi-valve resonance diaphragm, wherein the bonding layer surrounds the outer periphery of the vibration portion, and the quasi-valve resonance diaphragm is connected to the piezoelectric vibration diaphragm by using the bonding layer; and
the micro piezoelectric pump further comprises at least one support structure, wherein the support structure is located between the quasi-valve resonance diaphragm and the plate portion, so that a spacing is formed between the piezoelectric vibration diaphragm and the quasi-valve resonance diaphragm, and the at least one support structure is located on an inner side of the bonding layer.

22. The micro piezoelectric pump according to claim 21, wherein the support structure is protruded on a surface that is of the quasi-valve resonance diaphragm and that faces the piezoelectric vibration diaphragm, and the piezoelectric vibration diaphragm abuts against the support structure.

23. The micro piezoelectric pump according to claim 21 or 22, wherein the support structure is in a ring shape, each support structure comprises a plurality of support parts, and the plurality of support parts are spaced from each other and evenly disposed in an enclosing manner, to form the ring shape.

24. The micro piezoelectric pump according to claim 21 or 22, wherein the support structure is in a closed ring shape.

25. The micro piezoelectric pump according to claim 21 or 22, wherein the quasi-valve resonance diaphragm is provided with at least two support structures; and
in two adjacent support structures, one support structure is located on an inner side of the other support structure, there is a gap between the two adjacent support structures, and the bonding layer is located in the gap.

26. The micro piezoelectric pump according to claim 23, wherein the support part comprises at least two sub-support bodies, and two adjacent sub-support bodies are spaced from each other.

27. The micro piezoelectric pump according to claim 23, wherein a cross-sectional shape of the support part comprises at least a long strip shape, a cylindrical shape, an arc shape, and a square shape.

28. The micro piezoelectric pump according to claim 21 or 22, wherein the support structure comprises a plating layer.

29. The micro piezoelectric pump according to claim 21 or 22, wherein the support structure comprises a metal wire.

30. The micro piezoelectric pump according to claim 21 or 22, wherein a height range of the support structure is 5 µm to 12 µm.

31. The micro piezoelectric pump according to claim 21 or 22, further comprising an air intake layer and an air intake channel that are stacked, wherein the air intake channel is located between the air intake layer and the quasi-valve resonance diaphragm; and
an air intake hole is provided on the air intake layer, and the air intake hole communicates with the air intake opening through the air intake channel.

32. The micro piezoelectric pump according to claim 21 or 22, further comprising a front chamber structure layer, an air leakage diaphragm, an air vent channel, and an air vent layer that are sequentially stacked, wherein the piezoelectric vibration diaphragm is located between the quasi-valve resonance diaphragm and the front chamber structure layer; and
the air discharge opening communicates with the front chamber structure layer, the front chamber structure layer communicates with the air leakage diaphragm, the air leakage diaphragm communicates with the air vent layer through the air vent channel, and an air vent hole is provided on the air vent layer.

33. An electronic device, comprising at least a housing, an air bag, and the micro piezoelectric pump according to any one of claims 21 to 32, wherein
the air bag and the micro piezoelectric pump are disposed in the housing, and the micro piezoelectric pump communicates with the air bag.

34. The electronic device according to claim 33, wherein the housing comprises a wristband, and the air bag is disposed in the wristband.
